# EUROPEAN PATENT APPLICATION

(11) **EP 4 632 073 A1**
(43) Date of publication of application: **15.10.2025**
(21) Application number: 23903988.6
(22) Date of filing: 13.12.2023
(51) Int. Cl.: C12N 15/81, C12N 9/10, C12P 23/00

(54) **MICROORGANISM HAVING WEAKENED CHITIN TRANSGLYCOSYLASE ACTIVITY AND ABILITY TO PRODUCE RETINOIDS, AND METHOD FOR PRODUCING RETINOIDS BY USING SAME**

(30) Priority: 13.12.2022 KR 20220174083
(71) Applicant: CJ CheilJedang Corporation, Seoul 04560 (KR)
(72) Inventor: PARK, Hye Min, Seoul 04560 (KR); LEE, Peter, Seoul 04560 (KR); LEE, Dong Pil, Seoul 04560 (KR); KIM, Jae Eung, Seoul 04560 (KR)
(74) Representative: Meissner Bolte Partnerschaft mbB
(86) International application number: PCT/KR2023/020521
(87) International publication number: WO 2024/128787

(57) **Abstract**

Provided are a microorganism of the *Yarrowia* sp. having a retinoid production ability, in which the activity of chitin transglycosylase is weakened, a method of producing retinoids using the same, a composition for producing retinoids, use in producing retinoids, and a method of preparing the microorganism.

## Description

### [Technical Field]

The present disclosure relates to a microorganism of the *Yarrowia* sp. having a retinoid production ability, in which the activity of chitin transglycosylase is weakened, a method of producing retinoids using the same, a composition for producing retinoids, use in producing retinoids, and a method of preparing the microorganism.

### [Background Art]

Retinol, which is a fat-soluble vitamin, is an essential vitamin involved in eye health of improving nyctalopia, immune enhancement, skin health, etc. Currently, retinol is being produced and sold through chemical synthesis methods mainly by global advanced companies, but studies are being conducted to produce retinol based on microbial fermentation.

To this end, many technologies have been developed to stabilize the retinol compound itself in compositions or products comprising retinol (US Patent No. 6858217). However, the development of methods for stably increasing retinol production remains insignificant.

### [Disclosure]

### [Technical Problem]

A problem to be solved in the present disclosure is to provide a microorganism of the *Yarrowia* sp. having a retinoid production ability, in which the activity of chitin transglycosylase is weakened, a method of producing retinoids using the same, a composition for producing retinoids, use in producing retinoids, and a method of preparing the microorganism.

### [Technical Solution]

The present disclosure provides a microorganism of the *Yarrowia* sp. having a retinoid production ability, in which the activity of chitin transglycosylase is weakened.

The present disclosure provides a method of producing retinoids, the method comprising the step of culturing the microorganism of the present disclosure in a medium.

The present disclosure provides a method of preparing a microorganism having a retinoid production ability, the method comprising the step of weakening the activity of chitin transglycosylase in a microorganism of the *Yarrowia* sp. having a retinoid production ability.

The present disclosure provides a method of increasing excretion of retinoids, the method comprising the step of weakening the activity of chitin transglycosylase in a microorganism of the *Yarrowia* sp. having a retinoid production ability.

The present disclosure provides a composition for producing retinoids, the composition comprising the microorganism of the present disclosure or a culture thereof.

The present disclosure provides use of the microorganism of the present disclosure in producing retinoids.

### [Advantageous Effects]

Retinoids may be produced by using a microorganism of the present disclosure.

### [Brief Description of the Drawing]

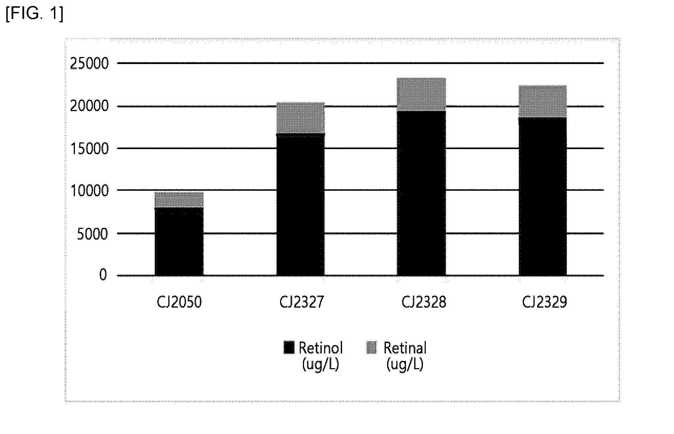

FIG. 1 shows retinoid concentrations in a flask culture test of a control group (CC08-2050) and chitin transglycosylase-weakened microorganisms (CJ2327, CJ2328, and CJ2329).

### [Detailed Description of Preferred Embodiments]

The present disclosure will be described in detail as follows. Meanwhile, each description and embodiment disclosed in this disclosure may also be applied to other descriptions and embodiments. That is, all combinations of various elements disclosed in this disclosure fall within the scope of the present disclosure. Further, the scope of the present disclosure is not limited by the specific description described below. Further, a number of papers and patent documents are referenced and cited throughout this specification. The disclosures of the cited papers and patent documents are incorporated herein by reference in their entirety to further clarify the level and scope of the subject matter to which the present disclosure pertains.

An aspect of the present disclosure provides a microorganism of the *Yarrowia* sp. having a retinoid production ability, in which the activity of chitin transglycosylase is weakened.

The "chitin transglycosylase" of the present disclosure is an enzyme that is able to catalyze the transfer of chitin to beta(1-6) glucan and beta(1-3) glucan in the cell wall.

The chitin transglycosylase of the present disclosure may be CRH1 protein, CRH2 protein, or a combination thereof. For example, the combination of CRH1 protein or CRH2 protein may comprise a combination of CRH1 protein and CRH2 protein. Further, since two or more CRH1 proteins having different amino acid sequences or two or more CRH2 proteins having different amino acid sequences may exist, the combination of CRH1 protein or CRH2 protein may comprise a combination of one or more CRH1 proteins (e.g., a combination of CRH1 having a specific amino acid sequence and CRH1 having an amino acid sequence different therefrom), a combination of CRH2 proteins (e.g., a combination of CRH2 having a specific amino acid sequence and CRH2 having an amino acid sequence different therefrom), and a combination of one or more CRH1 proteins and one or more CRH2 proteins (e.g., a combination of CRH1 having a specific amino acid sequence, CRH1 having an amino acid sequence different therefrom, and CRH2 having an amino acid sequence different therefrom), etc.

With respect to the objects of the present disclosure, the chitin transglycosylase may comprise any one as long as it is able to enhance the retinoid production ability and/or excretion ability. For example, the increase in the retinoid production ability may be due to, but is not limited to, an increase in the retinoid excretion ability.

In one embodiment, in a microorganism of the *Yarrowia* sp., the activity of chitin transglycosylase of the present disclosure is weakened, as compared to that of the endogenous or wild-type chitin transglycosylase, thereby increasing the retinoid production ability and/or excretion ability of the microorganism.

In one embodiment, the chitin transglycosylase of the present disclosure may comprise, have, or consist of SEQ ID NO: 1, SEQ ID NO: 2, SEQ ID NO: 3, or an amino acid sequence having 60% or more homology or identity to any one or more thereof, or may essentially consist of the amino acid sequence. The chitin transglycosylase of the present disclosure may be a polypeptide having the activity of chitin transglycosylase, together with the above sequence, but is not limited thereto.

For example, the amino acid sequence of the chitin transglycosylase of the present disclosure may be encoded by a *CRH1* gene, a *CRH2* gene, or a combination thereof, and for example, encoded by a CRH1 (YALI0C09680) gene, a CRH1 (YALI0E24673) gene, a CRH2 (YALI0B15510) gene, or a combination thereof, but is not limited thereto. The amino acid sequence may be obtained from various databases, such as NCBI's Genbank, etc., which is a known database, but is not limited thereto.

In one embodiment, the chitin transglycosylase of the present disclosure may be derived from *Yarrowia lipolytica,* but is not limited thereto.

In one embodiment, SEQ ID NO: 1 may be a CRH1(YALI0C09680) protein, SEQ ID NO: 2 may be a CRH1 (YALI0E24673) protein, and SEQ ID NO: 3 may be a CRH2 (YALI0B15510) protein.

Further, one embodiment of the chitin transglycosylase of the present disclosure may be described as a protein comprising SEQ ID NO: 1, SEQ ID NO: 2, SEQ ID NO: 3, or a combination thereof, but it is apparent to those skilled in the art that addition of a meaningless sequence upstream or downstream of the amino acid sequence of SEQ ID NO: 1, SEQ ID NO: 2, SEQ ID NO: 3, or a combination thereof, a naturally occurring mutation, or a silent mutation thereof is not excluded, and as long as a protein has an activity identical or corresponding to the activity of the protein comprising the amino acid sequence, it may belong to the chitin transglycosylase of the present disclosure.

For example, the chitin transglycosylase of the present disclosure may comprise the amino acid sequence of SEQ ID NO: 1, SEQ ID NO: 2, SEQ ID NO: 3, or a combination thereof, or may comprise an amino acid sequence having at least 60% or more, 62% or more, 63% or more, 64% or more, 62% or more, 65% or more, 70% or more, 75% or more, 76% or more, 77% or more, 78% or more, 80% or more, 85% or more, 86% or more, 87% or more, 88% or more, 89% or more, 90% or more, 91% or more, 92% or more, 93% or more, 94% or more, 95% or more, 96% or more, 97% or more, 98% or more or 99% or more homology or identity to the amino acid sequence of SEQ ID NO: 1, SEQ ID NO: 2, SEQ ID NO: 3, or a combination thereof. It is also apparent that a protein having an amino acid sequence in which part of the sequence is deleted, modified, substituted, or added also falls within the scope of the present disclosure as long as the amino acid sequence has such homology or identity and exhibits efficacy corresponding to that of the protein.

In the present disclosure, although it is described as 'a polypeptide or protein comprising an amino acid sequence described by a specific sequence number', 'a polypeptide or protein consisting of an amino acid sequence described by a specific sequence number', or a 'polypeptide or protein having an amino acid sequence described by a specific sequence number', it is apparent that any protein having an amino acid sequence in which part of the sequence is deleted, modified, substituted, conservatively substituted, or added may be used in the present disclosure if it has identical or corresponding activity to that of the polypeptide or protein consisting of the amino acid sequence of the corresponding sequence number. For example, it may be a case where the N-terminus and/or C-terminus of the amino acid sequence is added with a sequence that does not alter the function of the protein, a naturally occurring mutation, a silent mutation thereof, or a conservative substitution.

The "conservative substitution" refers to the substitution of an amino acid with another amino acid having similar structural and/or chemical properties. Such amino acid substitution may generally occur based on similarity of polarity, charge, solubility, hydrophobicity, hydrophilicity, and/or the amphipathic nature of a residue. Usually, a conservative substitution may hardly affect or may not affect activity of proteins.

As used herein, the term 'homology' or 'identity' means the degree of identity or similarity between two given amino acid sequences or base sequences and may be expressed as a percentage. The terms 'homology and identity' may often be used interchangeably with each other.

The sequence homology or identity of a conserved polynucleotide or polypeptide (comprising protein) is determined by standard alignment algorithms, and the default gap penalty established by a program to be used may be used together. Substantially, homologous or identical sequences may generally hybridize under moderately or highly stringent conditions to the full length of the sequence or at least about 50%, about 60%, about 70%, about 80%, or about 90% of the full-length. It is apparent that hybridization also comprises hybridization of a polynucleotide with a polynucleotide comprising a general codon or a codon in consideration of codon degeneracy.

Whether any two polynucleotide or polypeptide (comprising protein) sequences have homology, similarity, or identity may be determined using known computer algorithms such as the "FASTA" program, for example, using default parameters as in Pearson et al (1988) [Proc. Natl. Acad. Sci. USA 85]: 2444. Alternatively, it may be determined by the Needleman Wunsch algorithm (Needleman and Wunsch, 1970, J. Mol. Biol. 48: 443-453), as performed using the Needleman program of the EMBOSS package (EMBOSS: The European Molecular Biology Open Software Suite, Rice et al., 2000, Trends Genet. 16: 276-277) (version 5.0.0 or later) (GCG program package (Devereux, J., et al, Nucleic Acids Research 12: 387 (1984)), BLASTP, BLASTN, FASTA (Atschul, [S.] [F.,] [ET AL, J MOLEC BIOL 215]: 403 (1990); Guide to Huge Computers, Martin J. Bishop, [ED.,] Academic Press, San Diego,1994, and [CARILLO ETA/.](1988) SIAM J Applied Math 48: 1073). For example, homology, similarity, or identity may be determined using BLAST or ClustalW from the National Center for Biotechnology Information.

The homology, similarity, or identity of polynucleotides or polypeptides (comprising proteins) may, for example, be determined by comparing sequence information using, for example, the GAP computer program, such as Needleman et al. (1970), J Mol Biol. 48:443, as disclosed in Smith and Waterman, Adv. Appl. Math (1981) 2:482. Briefly, the GAP program defines the homology, similarity, or identity as the value obtained by dividing the number of similarly aligned symbols (i.e., nucleotides or amino acids) by the total number of symbols in the shorter of the two sequences. Default parameters for the GAP program may comprise (1) a binary comparison matrix (comprising a value of 1 for identities and 0 for non-identities) and the weighted comparison matrix of Gribskov et al(1986) Nucl. Acids Res. 14: 6745, as disclosed in Schwartz and Dayhoff, eds., Atlas Of Protein Sequence And Structure, National Biomedical Research Foundation, pp. 353-358 (1979) (or EDNAFULL substitution matrix (EMBOSS version of NCBI NUC4.4)); (2) a penalty of 3.0 for each gap and an additional 0.10 penalty for each symbol in each gap (or a gap opening penalty of 10 and a gap extension penalty of 0.5); and (3) no penalty for end gaps.

Further, whether any two polynucleotide or polypeptide (comprising protein) sequences have homology, similarity, or identity with each other may be identified by comparing the sequences in a Southern hybridization experiment under stringent conditions as defined, and appropriate hybridization conditions defined are within the skill of the art, and may be determined by a method well known to those skilled in the art (e.g., J. Sambrook et al., Molecular Cloning, A Laboratory Manual, 2nd Edition, Cold Spring Harbor Laboratory press, Cold Spring Harbor, New York, 1989; F.M. Ausubel et al., Current Protocols in Molecular Biology, John Wiley & Sons, Inc., New York).

As used herein, the term "polynucleotide" is a DNA strand having a certain length or more as a polymer of nucleotides in which nucleotide monomers are connected in a long chain by covalent bonds.

The polynucleotide sequence encoding the chitin transglycosylase of the present disclosure may be referred to as a CRH1 gene, a CRH2 gene, or a combination thereof, in one embodiment, as a CRH1 (YALI0C09680) gene, a CRH1 (YALI0E24673) gene, CRH2 (YALI0B15510) gene, or a combination thereof, which may comprise a polynucleotide sequence encoding the amino acid sequence represented by SEQ ID NO: 1, SEQ ID NO: 2, and/or SEQ ID NO: 3.

The polynucleotide may undergo various modifications in the coding region within the scope that does not change the amino acid sequence of the polypeptide or protein, due to codon degeneracy or in consideration of the codons preferred in an organism in which the polypeptide or protein is to be expressed. Specifically, the polynucleotide may comprise, consist of, or essentially consist of SEQ ID NO: 4, SEQ ID NO: 5, SEQ ID NO: 6, or a polynucleotide sequence having 60% or more homology or identity thereto, but is not limited thereto. For example, the polynucleotide may consist of a nucleotide sequence having 60% or more, 62% or more, 63% or more, 64% or more, 62% or more, 65% or more, 70% or more, 76% or more, 77% or more, 78% or more, 80% or more, 85% or more, 86% or more, 87% or more, 88% or more, 89% or more, 90% or more, 91% or more, 95% or more, 96% or more, 97% or more, 98% or more or 99% or more homology or identity to SEQ ID NO: 4, SEQ ID NO: 5, or SEQ ID NO: 6, but is not limited thereto.

Further, the polynucleotide of the present disclosure may comprise a probe, for example, any sequence without limitation as long as it is a sequence that is able to hybridize with a complementary sequence to the entirety or a part of the polynucleotide sequence under stringent conditions. The "stringent conditions" refer to conditions that enable specific hybridization between polynucleotides. These conditions are specifically described in documents (e.g., J. Sambrook et al., supra). For example, the stringent conditions may comprise conditions under which polynucleotides having a high homology or identity of 40% or more, specifically 90% or more, more specifically 95% or more, 96% or more, 97% or more, 98% or more, even more specifically 99% or more are hybridized with each other and polynucleotides having a homology or identity lower than the above homologies or identities are not hybridized with each other, or washing conditions of the common Southern hybridization, that is, washing once, specifically, twice or three times at a salt concentration and a temperature corresponding to 60°C, 1XSSC, 0.1% SDS, specifically 60°C, 0.1XSSC, 0.1% SDS, more specifically 68°C, 0.1XSSC, 0.1% SDS.

Hybridization requires that two nucleic acids comprise complementary sequences, although mismatches between bases are possible depending on the stringency of the hybridization. The term "complementary" is used to describe the relationship between nucleotide bases that may hybridize with each other. For example, with respect to DNA, adenine is complementary to thymine, and cytosine is complementary to guanine. Therefore, the polynucleotide of the present disclosure may comprise isolated nucleic acid fragments complementary to the entire sequence as well as nucleic acid sequences substantially similar thereto.

Specifically, polynucleotides having a homology or identity may be detected using hybridization conditions comprising a hybridization step at a Tm value of 55°C under the above-described conditions. Further, the Tm value may be 60°C, 63°C, or 65°C, but is not limited thereto, and may be appropriately adjusted by those skilled in the art depending on the purpose thereof.

The appropriate stringency for hybridizing the polynucleotides depends on the length of the polynucleotides and the degree of complementation, and these variables are well known in the art (J. Sambrook et al., supra).

As used herein, the term "vector" refers to a DNA construct for inserting a desired nucleotide sequence into the chromosome of a host, or a DNA construct comprising the nucleotide sequence of the polynucleotide encoding the desired polypeptide or protein operably linked to a suitable expression regulatory region (expression regulatory sequence) so as to be able to express the desired polypeptide or protein in a suitable host. The expression regulatory region may comprise a promoter capable of initiating transcription, any operator sequence for regulating transcription, a sequence encoding a suitable mRNA ribosome-binding site, and a sequence for regulating the termination of transcription and translation. Once transformed into a suitable host cell, the vector may replicate or function independently of the host genome, or may integrate into the genome thereof.

The vector of the present disclosure may be an insertion vector for inserting, into chromosome, the polynucleotide for weakening the activity of the chitin transglycosylase of the present disclosure, but is not limited thereto. The insertion of the polynucleotide into the chromosome may be performed by any method known in the art, for example, by homologous recombination, but is not limited thereto. The vector may further comprise a selection marker to confirm the insertion into the chromosome. The selection marker is for selecting the cells transformed with the vector, that is, for confirming whether the target nucleic acid molecule has been inserted, and markers that provide selectable phenotypes, such as drug resistance, auxotrophy, resistance to cytotoxic agents, or expression of surface polypeptides or proteins, may be used. Only cells expressing the selection marker are able to survive or show different phenotypes under the environment treated with the selective agent, and thus the transformed cells may be selected. The insertion vector may not comprise an origin of replication necessary for replication within the transformed cells.

The vector to be used in the present disclosure is not particularly limited, and any vector known in the art may be used. Examples of vectors commonly used may comprise natural or recombinant plasmids, cosmids, viruses, and bacteriophages. For example, as a phage vector or cosmid vector, pWE15, M13, MBL3, MBL4, IXII, ASHII, APII, t10, t11, Charon4A, and Charon21A, etc. may be used, and as a plasmid vector, those based on pDZ, pBR, pUC, pBluescriptll, pGEM, pTZ, pCL and pET, etc. may be used. Specifically, pDZ, pDC, pDCM2, pACYC177, pACYC184, pCL, pECCG117, pUC19, pBR322, pMW118, pCC1BAC vector, etc. may be used.

As used herein, the term "transformation" means that a vector comprising a desired polynucleotide (comprising an insertion vector for inserting, into chromosome, the polynucleotide for weakening the activity of chitin transglycosylase of the present disclosure) is introduced into a host cell to change the genetic traits of the host cell. The transformed polynucleotide may be inserted into the chromosome of the host cell or may be located outside the chromosome. Further, the polynucleotide may comprise DNA and/or RNA encoding the desired protein. The polynucleotide may be introduced in an appropriate form depending on the purpose of introduction. For example, the polynucleotide for expressing the desired protein may be introduced into a host cell in the form of an expression cassette, which is a gene construct comprising all elements required for self-expression. The expression cassette may usually comprise a promoter operably linked to the polynucleotide, a transcription termination signal, a ribosome binding site, and a translation termination signal. The expression cassette may be in the form of an expression vector capable of self-replicating. Further, the polynucleotide may be introduced into a host cell in its own form and operably linked to a sequence required for expression in the host cell, but is not limited thereto.

Further, as used herein, the term "operably linked" means that the polynucleotide sequence is functionally linked to a promoter sequence that initiates and mediates transcription of the downstream polynucleotide.

**The** method of transforming the vector of the present disclosure comprises any method of introducing a nucleic acid into a cell, and may be performed by selecting a suitable standard technique known in the art according to the host cell. For example, the transformation method may comprise electroporation, calcium phosphate (CaPO₄) precipitation, calcium chloride (CaCl₂) precipitation, microinjection, a polyethylene glycol (PEG) technique, a DEAE-dextran technique, a cationic liposome technique, a lithium acetate-DMSO technique, etc., but is not limited thereto.

As used herein, the term "microorganism" or "strain" comprises all of wild-type microorganisms or naturally or artificially genetically modified microorganisms, and it may be a microorganism in which a specific mechanism is weakened or strengthened due to the insertion of a foreign gene or an activity enhancement or inactivation of an endogenous gene, etc., and it may be a microorganism comprising a genetic modification for the production of a desired polypeptide, protein, or product.

The microorganism of the present disclosure may be a microorganism having a retinoid production ability. The 'microorganism having a retinoid production ability' may be used interchangeably with a 'microorganism producing retinoid'.

The microorganism of the present disclosure may be a microorganism into which polynucleotides encoding lycopene cyclase/phytoene synthase (crtYB) and phytoene desaturase (crtl) proteins are introduced to exhibit the activities of the proteins or to have the enhanced activities of the proteins such that a microorganism endogenously not having the retinoid production ability has the retinoid production ability, or the retinoid production ability of a microorganism having the retinoid production ability is further enhanced. The lycopene cyclase/phytoene synthase or phytoene desaturase may be a protein derived from *Xanthophyllomyces dendrorhous,* but is not limited thereto, as long as it is a protein exhibiting activity identical or similar thereto. In a specific embodiment, the lycopene cyclase/phytoene synthase or phytoene desaturase may consist of or may comprise an amino acid sequence of SEQ ID NO: 7 or SEQ ID NO: 8, respectively, but may consist of or may comprise an amino acid sequence having at least 60%, 65%, 70%, 75%, 80%, 85%, 86%, 87%, 88%, 89%, 90%, 91%, 92%, 93%, 94%, 95%, 96%, 97%, 98%, or 99% homology or identity to the above amino acid sequence while exhibiting activity corresponding to that of the lycopene cyclase/phytoene synthase or phytoene desaturase. It is also apparent that a protein in which part of the sequence is deleted, modified, substituted, or added also falls within the lycopene cyclase/phytoene synthase or phytoene desaturase as long as it has such a homology or identity and exhibits activity corresponding to that of the lycopene cyclase/phytoene synthase or phytoene desaturase. Further, in a specific embodiment, the polynucleotide encoding the lycopene cyclase/phytoene synthase or phytoene desaturase may consist of or may comprise a sequence of SEQ ID NO: 9 or SEQ ID NO: 10, respectively. The polynucleotide may undergo various modifications in the coding region within the scope that does not change the amino acid sequence, due to codon degeneracy or in consideration of the codons preferred in the microorganism of the present disclosure. Specifically, the polynucleotide may consist of or may comprise a nucleotide sequence having 60% or more, 70% or more, 80% or more, 85% or more, 90% or more, 95% or more, 96% or more, 97% or more, 98% or more, and less than 100% homology or identity to the sequence of SEQ ID NO: 9 or SEQ ID NO: 10, but is not limited thereto.

Further, the microorganism of the present disclosure may be a microorganism into which a polynucleotide encoding beta-carotene 15,15'-oxygenase (BLH) protein is introduced to exhibit the beta-carotene 15, 15'-oxygenase activity or to have the enhanced beta-carotene 15, 15'-oxygenase activity such that a microorganism endogenously not having the retinoid production ability has the retinoid production ability, or the retinoid production ability of a microorganism having the retinoid production ability is further enhanced. The beta-carotene 15, 15'-oxygenase may be a protein derived from uncultured marine bacterium 66A03, but is not limited thereto, as long as it is a protein exhibiting activity identical or similar thereto. In a specific embodiment, the beta-carotene 15, 15'-oxygenase may consist of or may comprise an amino acid sequence of SEQ ID NO: 11, but may consist of or may comprise an amino acid sequence having at least 60%, 65%, 70%, 75%, 80%, 85%, 86%, 87%, 88%, 89%, 90%, 91%, 92%, 93%, 94%, 95%, 96%, 97%, 98% or 99% homology or identity to the above amino acid sequence while exhibiting activity corresponding to that of the beta-carotene 15, 15'-oxygenase. It is also apparent that a protein in which part of the sequence is deleted, modified, substituted, or added also falls within the beta-carotene 15, 15'-oxygenase as long as it has such a homology or identity and exhibits activity corresponding to that of the beta-carotene 15, 15'-oxygenase. Further, in a specific embodiment, the polynucleotide encoding the beta-carotene 15, 15'-oxygenase may consist of or may comprise a sequence of SEQ ID NO: 12. The polynucleotide may undergo various modifications in the coding region within the scope that does not change the amino acid sequence, due to codon degeneracy or in consideration of the codons preferred in the microorganism of the present disclosure. Specifically, the polynucleotide may consist of or may comprise a nucleotide sequence having 60% or more, 70% or more, 80% or more, 85% or more, 90% or more, 95% or more, 96% or more, 97% or more, 98% or more, and less than 100% homology or identity to the sequence of SEQ ID NO: 12, but is not limited thereto.

The microorganism of the present disclosure may be a microorganism in which the activity of chitin transglycosylase is weakened to increase the retinoid production ability and/or excretion ability.

In the microorganism of the present disclosure, the increase in the retinoid production ability may be due to the increase in the retinoid excretion ability, but is not limited thereto.

The microorganism of the present disclosure may selectively excrete retinoids, but is not limited thereto.

The microorganism of the present disclosure may have the increased retinoid excretion ability, as compared to a microorganism having the retinoid production ability, in which the activity of chitin transglycosylase is not weakened, but is not limited thereto.

In one embodiment, the microorganism of the present disclosure may excrete retinoids selectively from beta-carotenes and retinoids.

The microorganism of the present disclosure may be obtained by additionally weakening the chitin transglycosylase of the present disclosure in a microorganism naturally having the chitin transglycosylase or retinoid production ability or a parent strain having the chitin transglycosylase or retinoid production ability.

For example, the microorganism of the present disclosure may comprise all microorganisms capable of producing retinoids, in which the chitin transglycosylase of the present disclosure is weakened.

The microorganism of the present disclosure may have the increased retinoid excretion ability, as compared to a microorganism having the retinoid production ability, in which the activity of chitin transglycosylase is not weakened.

For example, the microorganism of the present disclosure may be a recombinant strain in which the retinoid production ability and/or excretion ability are/is increased by weakening the activity of the chitin transglycosylase of the present disclosure in a natural wild-type microorganism, a microorganism having the retinoid production ability, and/or a microorganism comprising chitin transglycosylase. The recombinant strain in which the retinoid production ability and/or excretion ability are/is increased may be a microorganism in which the retinoid production ability and/or excretion ability are/is increased, as compared to a natural wild-type microorganism or a microorganism in which the activity of the chitin transglycosylase of the present disclosure is not weakened, but is not limited thereto.

For example, the microorganism in which the activity of the chitin transglycosylase of the present disclosure is not weakened, which is a target strain for comparing whether or not the retinoid production ability and excretion ability are increased, may be CC08-2050 (KCCM13294P, Ref. Park et al., Metabolic engineering 2022;73:26-37), but is not limited thereto. The strain designation of the CJ2050 strain described in the above reference (Park et al., Metabolic engineering 2022;73:26-37) is the CC08-2050 strain of the present disclosure, and the CC08-2050 strain of the present disclosure and the CJ2050 strain are identical strains.

For example, the recombinant strain and microorganism in which the retinoid production ability and/or excretion ability is/are increased may have an increased retinoid production ability and/or excretion ability of about 1% or more, about 2% or more, about 5% or more, about 7% or more, about 10% or more, about 20% or more, about 30% or more, about 40% or more, about 50% or more, about 60% or more, about 70% or more, about 80% or more, about 90% or more, or about 100% or more (the upper limit is not particularly limited, but may be, for example, about 200% or less), in another example, a retinoid production ability and/or excretion ability of about 1.01 times or more, about 1.02 times or more, about 1.05 times or more, about 1.07 times or more, about 1.1 times or more, about 1.2 times or more, about 1.3 times or more, about 1.4 times or more, about 1.5 times or more, about 1.6 times or more, about 1.7 times or more, about 1.8 times or more, about 1.9 times or more, or about 2 times or more (the upper limit is not particularly limited, but may be, for example, about 10 times or less), as compared to the parent strain before modification or the unmodified microorganism, but the increased amount is not limited thereto as long as the ability has an increased amount of a + value, as compared to the production ability and/or excretion ability of the parent strain before modification or the unmodified microorganism. The term "about" refers to a range comprising ±0.5, ±0.4, ±0.3, ±0.2, ±0.1, etc., and thus comprises all of the values in the range equivalent or similar to those stated after the term "about", but is not limited thereto.

For example, the recombinant strain or microorganism may have the reduced activity of chitin transglycosylase, as compared to the parent strain before modification or unmodified microorganism, and when the activity of endogenous chitin transglycosylase in the parent strain before modification or unmodified microorganism is regarded as 100%, the activity of chitin transglycosylase in the recombinant strain or microorganism may be reduced to 100% or less (e.g., reduced to 99.5%, 99%, 98%, 95%, 90%, 85%, 80%, etc.), but is not limited thereto.

As used herein, the term "unmodified microorganism" does not exclude strains comprising mutations that may occur naturally in microorganisms, and may be a wild-type strain or a natural strain itself or may be a strain before the trait is changed by genetic variation due to natural or artificial factors. For example, the unmodified microorganism may refer to a strain in which the chitin transglycosylase of the present disclosure is not weakened or before the chitin transglycosylase of the present disclosure is weakened. The "unmodified microorganism" may be used interchangeably with "strain before modification", "microorganism before modification", "unvaried strain", "unmodified strain", "unvaried microorganism", or "reference microorganism".

The microorganism of the present disclosure may be a microorganism of the *Yarrowia* sp., but is not limited thereto.

In one embodiment, the microorganism of the *Yarrowia* sp. of the present disclosure may be *Yarrowia lipolytica,* but is not limited thereto.

As used herein, the term "weakening" of the activity of chitin transglycosylase (comprising a polypeptide thereof and a protein thereof, the same applies hereinafter) is a concept comprising both cases where the activity is decreased, as compared to the endogenous activity, or the activity is absent. The weakening may be used interchangeably with terms such as inactivation, deficiency, deletion, down-regulation, decrease, reduce, attenuation, etc.

The weakening may also comprise a case where the activity of the chitin transglycosylase itself is decreased or eliminated due to variation of the polynucleotide encoding the chitin transglycosylase (comprising a polypeptide thereof and a protein thereof), etc., as compared to the activity of the chitin transglycosylase originally possessed by the microorganism, a case where the overall activity level and/or concentration (expression level) of the chitin transglycosylase in the cell is low due to inhibition of the expression of the gene of the polynucleotide encoding the same or by inhibition of translation into the chitin transglycosylase, as compared to that of the natural strain, a case where the polynucleotide is not expressed at all, and/or a case where the chitin transglycosylase activity is absent even when the polynucleotide is expressed.

The "endogenous activity" means the activity of a specific chitin transglycosylase (comprising a polypeptide thereof and a protein thereof) originally possessed by the parent strain before change of the trait, a wild-type or unmodified microorganism when the trait is changed by genetic variation due to natural or artificial factors. This may be used interchangeably with "activity before modification". The fact that the activity of the chitin transglycosylase is "weakened, inactivated, deficient, decreased, down-regulated, reduced, or attenuated" as compared to the endogenous activity means that the activity is lowered, as compared to the activity of a specific chitin transglycosylase originally possessed by the parent strain before change of the trait or the unmodified microorganism.

With respect to the objects of the present disclosure, the microorganism of the present disclosure may have the enhanced retinoid production ability and/or excretion ability due to the weakening of the activity of chitin transglycosylase.

Such weakening of the activity of chitin transglycosylase may be performed by any method known in the art, but the method is not limited thereto, and the weakening may be achieved by applying various methods well known in the art (e.g., Nakashima N et al., Bacterial cellular engineering by genome editing and gene silencing. Int J Mol Sci. 2014;15(2):2773-2793, Sambrook et al. Molecular Cloning 2012, etc.).

Specifically, the weakening of the activity of chitin transglycosylase in the present disclosure may be:
1) deletion of the entirety or a part of the gene encoding the chitin transglycosylase polypeptide;
2) modification of an expression regulatory region (or expression regulatory sequence) to decrease the expression of the gene encoding the chitin transglycosylase polypeptide;
3) modification of an amino acid sequence constituting the chitin transglycosylase polypeptide to eliminate or weaken the activity of chitin transglycosylase (e.g., deletion/substitution/addition of one or more amino acids in the amino acid sequence);
4) modification of a gene (comprising a polynucleotide) sequence encoding the chitin transglycosylase polypeptide to eliminate or weaken the activity of chitin transglycosylase (e.g., deletion/substitution/addition of one or more nucleic acid bases in the nucleic acid sequence of the gene of the polypeptide to encode the polypeptide that has been modified to eliminate or weaken the activity of the polypeptide);
5) modification of a start codon of a gene transcript encoding the chitin transglycosylase polypeptide or a nucleotide sequence encoding a Shine-Dalgarno sequence or **5'-UTR** region;
6) introduction of an antisense oligonucleotide (e.g., antisense RNA) that complementarily binds to the transcript of the gene encoding the chitin transglycosylase polypeptide;
7) addition of a sequence complementary to a Shine-Dalgarno sequence in front of the Shine-Dalgarno sequence of the gene encoding the chitin transglycosylase polypeptide in order to form a secondary structure to which ribosome cannot be attached;
8) addition of a promoter to be transcribed in the opposite direction to the 3' end of the open reading frame (ORF) of the gene sequence encoding the chitin transglycosylase polypeptide (reverse transcription engineering, RTE); or
9) a combination of two or more selected from 1) to 8), but is not particularly limited thereto.

For example,
1) the deletion of a part or the entirety of the gene encoding the polypeptide may be removal of the entire polynucleotide encoding the endogenous target polypeptide in the chromosome, replacement with a polynucleotide in which some nucleotides are deleted, or replacement with a marker gene.
2) The modification of the expression regulatory region (or expression regulatory sequence) may be occurrence of variation on the expression regulatory region (or expression regulatory sequence) due to deletion, insertion, non-conservative or conservative substitution, or a combination thereof, or replacement with a sequence exhibiting weaker activity. The expression regulatory region comprises a promoter, an operator sequence, a sequence encoding a ribosome binding site, and a sequence regulating the termination of transcription and translation, but is not limited thereto.
3) and 4) The modification of the amino acid sequence or polynucleotide sequence may be occurrence of variation on the sequence due to deletion, insertion, or non-conservative or conservative substitution of the amino acid sequence of the polypeptide or the polynucleotide sequence encoding the polypeptide, or a combination thereof, or replacement with an amino acid sequence or a polynucleotide sequence improved to have weaker activity or an amino acid sequence or a polynucleotide sequence improved to be inactive so that the activity of the polypeptide is weakened, but is not limited thereto. For example, expression of the gene may be inhibited or weakened by introducing variation into the polynucleotide sequence and forming a stop codon, but is not limited thereto.
5) The modification of a start codon of a gene transcript encoding the polypeptide or a nucleotide sequence encoding a 5'-UTR region may be, for example, substitution with a nucleotide sequence encoding another start codon having a lower polypeptide expression rate, as compared to an endogenous start codon, but is not limited thereto.
6) The introduction of an antisense oligonucleotide (e.g., antisense RNA) that complementarily binds to the transcript of the gene encoding the polypeptide, may refer to documents, for example, [Weintraub, H. et al., Antisense-RNA as a molecular tool for genetic analysis, Reviews - Trends in Genetics, Vol. 1(1) 1986].
7) The addition of a sequence complementary to a Shine-Dalgarno sequence in front of the Shine-Dalgarno sequence of the gene encoding the chitin transglycosylase polypeptide in order to form a secondary structure to which ribosome cannot be attached may be to make mRNA translation impossible or to slow down the mRNA translation rate.

Further, 8) the addition of a promoter to be transcribed in the opposite direction to the 3' end of the open reading frame (ORF) of the gene sequence encoding the polypeptide (reverse transcription engineering, RTE) may be to weaken the activity by making an antisense nucleotide complementary to the transcript of the gene encoding the polypeptide.

In one embodiment, in the microorganism of the present disclosure, any one or more of the genes encoding chitin transglycosylase may be deleted, but is not limited thereto.

As used herein, the term "retinoid" refers chemically to a group of vitamin A or chemical compounds chemically related thereto.

In one embodiment, the retinoid may be any one selected from the group consisting of retinol, retinal, retinoic acid, and retinyl ester, but is not limited thereto.

In one embodiment, retinol may be converted to other retinoid compounds (e.g., retinal, retinoic acid, and retinyl esters) by methods known in the art.

Another aspect of the present disclosure provides a method of producing retinoids, the method comprising the step of culturing the microorganism of the present disclosure in a medium.

The microorganism is as described in other aspects.

As used herein, the term "culturing" refers to growing the microorganism of the present disclosure under appropriately adjusted environmental conditions. In the present disclosure, the culture procedure may be performed according to appropriate media and culture conditions known in the art. Such a culturing process may be easily adjusted and used by a person skilled in the art according to the selected strain. Specifically, the culturing may be of a batch type, a continuous type, and/or a fed-batch type, but is not limited thereto.

The microorganism of the present disclosure may be cultured under aerobic conditions in a common medium comprising appropriate carbon sources, nitrogen sources, phosphorus sources, inorganic compounds, amino acids, and/or vitamins, etc., while controlling the temperature, pH, etc.

In the present disclosure, the carbon source may comprise carbohydrates, such as glucose, saccharose, lactose, fructose, sucrose, maltose, etc.; sugar alcohols, such as mannitol, sorbitol, etc.; organic acids, such as pyruvic acid, lactic acid, citric acid, etc.; and amino acids, such as glutamic acid, methionine, lysine, etc. In addition, natural organic nutrient sources may be used, such as starch hydrolysates, molasses, blackstrap molasses, rice bran, cassava, bagasse, and corn steep liquor, and specifically, carbohydrates such as glucose and sterile pretreated molasses (i.e., molasses converted to reduced sugars) may be used, and appropriate amounts of various other carbon sources may be used without limitation. These carbon sources may be used alone or in a combination of two or more thereof, but are not limited thereto.

As for the nitrogen sources, inorganic nitrogen sources, such as ammonia, ammonium sulfate, ammonium chloride, ammonium acetate, ammonium phosphate, ammonium carbonate, ammonium nitrate, etc.; amino acids, such as glutamic acid, methionine, glutamine, etc.; and organic nitrogen sources, such as peptone, NZ-amine, meat extracts, yeast extracts, malt extracts, corn steep liquor, casein hydrolysates, fishes or decomposition products thereof, defatted soybean cake or degradation products thereof, etc. may be used. These nitrogen sources may be used alone or in a combination of two or more thereof, but are not limited thereto.

The phosphorus sources may comprise potassium phosphate monobasic, potassium phosphate dibasic, and sodium-comprising salts corresponding thereto. As for inorganic compounds, sodium chloride, calcium chloride, iron chloride, magnesium sulfate, iron sulfate, manganese sulfate, calcium carbonate, etc. may be used, and in addition, amino acids, vitamins, and/or suitable precursors may be comprised. These constituent ingredients or precursors may be added to the medium in a batch or continuous manner. However, the present disclosure is not limited thereto.

During culturing of the microorganism of the present disclosure, pH of the medium may be adjusted by adding compounds, such as ammonium hydroxide, potassium hydroxide, ammonia, phosphoric acid, and sulfuric acid, etc., to the medium in an appropriate manner. In addition, an anti-foaming agent, such as a fatty acid polyglycol ester, may be added to suppress foam formation during culturing. In addition, oxygen or oxygen-comprising gas may be injected into the medium to maintain the aerobic state of the medium, or no gas may be injected or nitrogen, hydrogen or carbon dioxide gas may be injected to maintain the anaerobic or non-aerobic state of the medium, but is not limited thereto.

In addition, the culture medium may comprise metal salts such as magnesium sulfate or iron sulfate required for growth. Lastly, in addition to the above substances, essential growth substances such as amino acids and vitamins may be used. In addition, suitable precursors may be used in the culture medium. The above-mentioned raw materials may be added to the culture in a batch or continuous manner in an appropriate manner during the culture process, but are not limited thereto.

In the present disclosure, during culturing of the microorganism, pH of the culture may be adjusted by adding compounds, such as ammonium hydroxide, potassium hydroxide, ammonia, phosphoric acid, sulfuric acid, etc., to the culture in an appropriate manner. In addition, an anti-foaming agent, such as a fatty acid polyglycol ester, may be added to suppress foam formation during culturing. In addition, oxygen or oxygen-comprising gas may be injected into the culture to maintain the aerobic state of the culture, or no gas may be injected or nitrogen, hydrogen or carbon dioxide gas may be injected to maintain the anaerobic or non-aerobic state, but is not limited thereto.

In the culturing of the present disclosure, the culture temperature may be maintained at 20°C to 35°C, specifically 25°C to 35°C, and the culturing may be continued until the desired amount of a useful material is obtained, and may be carried out for about 10 hours to 160 hours, about 20 hours to 130 hours, about 24 hours to 120 hours, about 36 hours to 120 hours, about 48 hours to 120 hours, about 48 hours or more, or about 48 hours, about 72 hours, or about 120 hours, but is not limited thereto.

The method of producing retinoids of the present disclosure may further comprise the step of recovering retinoids from the microorganism or medium.

The desired retinoids may be recovered from the medium using a suitable method known in the art, depending on the method of culturing the microorganism of the present disclosure, for example, batch, continuous or fed-batch culture methods. For example, centrifugation, filtration, treatment with a crystallized protein precipitating agent (salting-out), extraction, sonication, ultrafiltration, dialysis, various types of chromatography, such as molecular sieve chromatography (gel filtration), adsorption chromatography, ion exchange chromatography, and affinity chromatography, HPLC, and a combination of these methods may be used, but are not limited thereto.

The method may further comprise an additional purification process. The purification process may be performed by using an appropriate method known in the art.

In one embodiment, in the method of producing retinoids of the present disclosure, the microorganism having the retinoid excretion ability, in which the activity of chitin transglycosylase is weakened, is used, and therefore, in the present disclosure, retinoids may be produced without using cell disruption of microorganisms or dodecane as a solvent, which is widely used in retinoid extraction, but is not limited thereto.

The method of producing retinoids of the present disclosure may further comprise the step of converting retinol, which is expressed by the microorganism of the present disclosure, into retinoid other than retinol. In the method of producing retinoids of the present disclosure, the converting step may be further comprised, after the culturing step or the recovering step. The converting step may be performed using a suitable method known in the art. For example, the conversion may be performed using retinol acyltransferase, but is not limited thereto.

In one embodiment, the retinoid other than the retinol may be any one selected from the group consisting of retinal, retinoic acid, and retinyl ester, but is not limited thereto as long as it is comprised in the retinoid.

Still another aspect of the present disclosure provides a method of preparing a microorganism of the *Yarrowia* sp. having the retinoid production ability, the method comprising the step of weakening the activity of chitin transglycosylase in the microorganism of the *Yarrowia* sp. having the retinoid production ability.

Still another aspect of the present disclosure provides a method of increasing retinoid excretion, the method comprising the step of weakening the activity of chitin transglycosylase in a microorganism of the *Yarrowia* sp. having the retinoid production ability.

The method of increasing retinoid excretion may be a method of increasing retinoid excretion in a microorganism of the *Yarrowia* sp. having the retinoid production ability.

The step of weakening the activity of chitin transglycosylase may be a step of modifying the microorganism of the *Yarrowia* sp. such that chitin transglycosylase is weakened, but it is as described in other aspects.

Still another aspect of the present disclosure provides a composition for producing retinoids, the composition comprising any one or more of the microorganisms of the *Yarrowia* sp., in which the activity of chitin transglycosylase is weakened, and a culture thereof.

The composition of the present disclosure may further comprise any appropriate excipient that is commonly used in the composition for producing retinoids, and such excipients may comprise, for example, a preserving agent, a wetting agent, a dispersing agent, a suspending agent, a buffer, a stabilizing agent, an isotonic agent, etc., but are not limited thereto.

The chitin transglycosylase, the microorganism of the *Yarrowia* sp., in which the activity thereof is weakened, the culture, the retinoids, etc. are as described in other aspects.

Still another aspect of the present disclosure provides use of the microorganism of the *Yarrowia* sp., in which the activity of chitin transglycosylase of the present disclosure is weakened, in producing retinoids.

The chitin transglycosylase, the microorganism of the *Yarrowia* sp., in which the activity thereof is weakened, the retinoids, etc. are as described in other aspects.

### [Mode for Carrying Out the Invention]

Hereinafter, the present disclosure will be described in more detail by way of exemplary embodiments. However, the following exemplary embodiments are only preferred embodiments for illustrating the present disclosure, and thus are not intended to limit the scope of the present disclosure thereto. Meanwhile, technical matters not described in the present specification may be sufficiently understood and easily implemented by those skilled in the technical field of the present disclosure or similar technical fields.

### Example 1. Weakening of CRH1 (YALI0C09680) of Yarrowia sp. Strain Producing Retinoid

To weaken endogenous CRH1 (YALI0C09680) of a retinoid-producing *Yarrowia* sp. strain CC08-2050 (Park et al., Metabolic engineering 2022;73:26-37), deposited as KCCM13294P, ORF of CRH1 (YALI0C09680) in the genome was deleted. To this end, an ORF sequence (SEQ ID NO: 4) of CRH1 (YALI0C09680) was obtained based on a nucleotide sequence registered in Kyoto Encyclopedia of Genes and Genomes (KEGG). In addition, a CRH1 (YALI0C09680) deletion cassette was constructed using primers as shown in Table 1 and URA3 gene (SEQ ID NO: 13) of Y. *lipolytica* as a selection marker. That is, PCR was performed using the genomic DNA of CC08-2050 as a template and primers of SEQ ID NO: 14 and SEQ ID NO: 15, SEQ ID NO: 16 and SEQ ID NO: 17, SEQ ID NO: 18 and SEQ ID NO: 19, and SEQ ID NO: 20 and SEQ ID NO: 21, respectively. PCR conditions were 35 cycles of denaturation at 95°C for 1 minute; annealing at 55°C for 1 minute; and polymerization at 72°C for 2 minutes. The resulting DNA fragments were produced into one cassette through overlap extension PCR.

The cassette thus constructed was introduced into the CC08-2050 strain by a heat shock method (D.-C. Chen et al., Appl Microbiol Biotechnol, 1997), and then colonies formed on a solid medium (YLMM1) without uracil were obtained. The colonies, in which insertion of the cassette into the genome was confirmed using primers of SEQ ID NO: 22 and SEQ ID NO: 23, were cultured on 5-FOA solid medium at 30°C for 3 days, and URA3 marker was recovered by obtaining colonies grown on the 5-FOA solid medium. The final strain thus obtained was named CJ2327.

**[Table 1]**

| SEQ ID NO. | Sequence (5'- 3') | PCR product |
|---|---|---|
| 14 | TCACCACCCATCACGCTCAATACCCCTGTG | Homology left arm |
| 15 | ATGACGAGTCAGACAGGAGGCATGCTGTGTGGGTTT TGTAGGTG | |
| 16 | CACCTACAAAACCCACACAGCATGCCTCCTGTCTGA CTCGTCAT | URA3 |
| 17 | GATGGATTTGGTGAGTGCGAGTTGGTGGTATTGTGA CTGGGGAT | |
| 18 | ATCCCCAGTCACAATACCACCAACTCGCACTCACCAA ATCCATC | Repeat region |
| 19 | GTAAAACACCCGTCAACTAGAATGCTGTGTGGGTTTT GTAGGTG | |
| 20 | CACCTACAAAACCCACACAGCATTCTAGTTGACGGGT GTTTTAC | Homology right arm |
| 21 | ATGCCACCCCGCTCAGCTTGGAACCTTCAC | |
| 22 | TATCTGGGGTATGACGGGAAACGG | Forward |
| 23 | AGCCCATTTTGTGCAACGACACAT | Reverse |

As the YLMM1 medium and 5-FOA medium described above, those having the following compositions were used.

### <Yarrowia lipolytica minimal media1 (YLMM1)>

20 g/L of glucose, 6.7 g/L of yeast nitrogen base without amino acids, 2 g/L of yeast synthetic drop-out medium supplements without uracil, 15 g/L of agar

### <5-Fluoroorotic acid (5-FOA)>

20 g/L of glucose, 6.7 g/L of yeast nitrogen base without amino acids, 2 g/L of yeast synthetic drop-out medium supplements without uracil, 50 µg/mL of uracil, 1 g/L of 5-fluoroorotic acid (5-FOA), 15 g/L of agar

### Example 2. Weakening of CRH1 (YALI0E24673) of Yarrowia sp. Strain Producing Retinoid

To weaken endogenous CRH1 (YALI0E24673) of a retinoid-producing *Yarrowia* sp. strain CC08-2050 (KCCM13294P), ORF of CRH1 (YALI0E24673) in the genome was deleted. To this end, an ORF sequence (SEQ ID NO: 5) of CRH1 (YALI0E24673) was obtained based on a nucleotide sequence registered in Kyoto Encyclopedia of Genes and Genomes (KEGG). In addition, a CRH1 (YALI0E24673) deletion cassette was constructed using primers as shown in Table 2 and URA3 gene (SEQ ID NO: 13) of *Y. lipolytica* as a selection marker. That is, PCR was performed using the genomic DNA of CC08-2050 as a template and primers of SEQ ID NO: 24 and SEQ ID NO: 25, SEQ ID NO: 26 and SEQ ID NO: 27, SEQ ID NO: 28 and SEQ ID NO: 29, and SEQ ID NO: 30 and SEQ ID NO: 31, respectively. PCR conditions were 35 cycles of denaturation at 95°C for 1 minute; annealing at 55°C for 1 minute; and polymerization at 72°C for 2 minutes. The resulting DNA fragments were produced into one cassette through overlap extension PCR.

**[Table 2]**

| SEQ ID NO. | Sequence (5'- 3') | PCR product |
|---|---|---|
| 24 | CTCTGACGCGGTTTAGAGCCTACCGAGGCG | Homology left arm |
| 25 | ATGACGAGTCAGACAGGAGGCAAATGATTAATGAAT GTGTACTA | |
| 26 | TAGTACACATTCATTAATCATTTGCCTCCTGTCTGACT CGTCAT | URA3 |
| 27 | GTGTCAAAAAAACGAGATCCATTGGTGGTATTGTGA CTGGGGAT | |
| 28 | ATCCCCAGTCACAATACCACCAATGGATCTCGTTTTT TTGACAC | Repeat region |
| 29 | AACAAATAATTATTCAATACAAAATGATTAATGAATGTG TACTA | |
| 30 | TAGTACACATTCATTAATCATTTTGTATTGAATAATTAT T ATCATGTGTAAGTAATTAGTAAAACATAACTTATTAATA A | Homology right arm |
| 31 | CACCGTGCACACCTCGTGGCTCCCTTGAAA | |
| 32 | TGTTTACTTACAAGTTGAATGGTC | Forward |
| 33 | AATCCACCTTGGCTGGGTGCTCTAC | Reverse |

The cassette thus constructed was introduced into the CC08-2050 strain by a heat shock method, and then colonies formed on a solid medium (YLMM1, a medium with the same composition as the YLMM1 medium in Example 1) without uracil were obtained. The colonies, in which insertion of the cassette into the genome was confirmed using primers of SEQ ID NO: 32 and SEQ ID NO: 33, were cultured on 5-FOA solid medium at 30°C for 3 days, and URA3 marker was recovered by obtaining colonies grown on the 5-FOA solid medium. The final strain thus obtained was named CJ2328.

### Example 3. Weakening of CRH2 (YALI0B15510) of Yarrowia sp. Strain Producing Retinoid

To weaken endogenous CRH2 (YALI0B15510) of a retinoid-producing *Yarrowia* sp. strain CC08-2050, ORF of CRH2 (YALI0B15510) was deleted. To this end, an ORF sequence (SEQ ID NO: 6) of CRH2 (YALI0B15510) was obtained based on a nucleotide sequence registered in Kyoto Encyclopedia of Genes and Genomes (KEGG). In addition, a CRH2 (YALI0B15510) deletion cassette was constructed using primers as shown in Table 3 and URA3 gene (SEQ ID NO: 13) of *Y. lipolytica* as a selection marker. That is, PCR was performed using the genomic DNA of CC08-2050 as a template and primers of SEQ ID NO: 34 and SEQ ID NO: 35, SEQ ID NO: 36 and SEQ ID NO: 37, SEQ ID NO: 38 and SEQ ID NO: 39, and SEQ ID NO: 40 and SEQ ID NO: 41, respectively. PCR conditions were 35 cycles of denaturation at 95°C for 1 minute; annealing at 55°C for 1 minute; and polymerization at 72°C for 2 minutes. The resulting DNA fragments were produced into one cassette through overlap extension PCR.

**[Table 3]**

| SEQ ID NO. | Sequence (5'- 3') | PCR product |
|---|---|---|
| 34 | CCGATTTTAAATTTCACTTACACTTCACTT | Homology left arm |
| 35 | ATGACGAGTCAGACAGGAGGCATTTGTACTGGGTG ATGCAAACG | |
| 36 | CGTTTGCATCACCCAGTACAAATGCCTCCTGTCTGA CTCGTCAT | URA3 |
| 37 | CTGTACTGGGACTCATCGGGGATGGTGGTATTGTG ACTGGGGAT | |
| 38 | atccccagtcacaataccaccaTCCCCGATGAGTCCCAGTAC AG | Repeat region |
| 39 | CGCTGCGAGGATATCACTTAGCTTTGTACTGGGTGA TGCAAACG | |
| 40 | CGTTTGCATCACCCAGTACAAAGCTAAGTGATATCC TCGCAGCG | Homology right arm |
| 41 | CTGGTACATGTAGGAGTTCTTTCGCCCCTG | |
| 42 | CGTTGTTATGGAGACGCGAAGTA | Forward |
| 43 | GTTCTGGTTATATACCGGAGAGTA | Reverse |

The cassette thus constructed was introduced into the CC08-2050 strain by a heat shock method, and then colonies formed on a solid medium (YLMM1) without uracil were obtained. The colonies, in which insertion of the cassette into the genome was confirmed using primers of SEQ ID NO: 42 and SEQ ID NO: 43, were cultured on 5-FOA solid medium at 30°C for 3 days, and URA3 marker was recovered by obtaining colonies grown on the 5-FOA solid medium. The final strain thus obtained was named CJ2329.

### Example 4. Comparative Evaluation of Retinoid Production and Excretion Abilities in Strains with Weakened CRH

To compare the retinoid production and excretion levels of the strains prepared through Examples 1 to 3, a flask test was conducted. The retinoid-producing *Yarrowia* strain (CC08-2050 (KCCM13294P); control group), CRH1 (YALI0C09680) deficient strain (CJ2327) prepared in Example 1, CRH1 (YALI0E24673) deficient strain (CJ2328), and CRH2 (YALI0B15510) deficient strain (CJ2329) were each inoculated into a 250 ml corner-baffle flask comprising 25 ml of YPDLU medium supplemented with 0.05% 3,5-di-tert-4-butylhydroxytoluene (BHT) at an initial OD = 2, and cultured under conditions of 30°C and 200 rpm. As the YPDLU medium described above, a medium having the following composition was used.

### <YPDLU>

40 g/L of glucose, 20 g/L of bacto peptone, 10 g/L of yeast extract, 1 g/L of uracil, 1 g/L of leucine, 100 ml/L of 1 M phosphate buffer (pH 7.0)

The growth of each strain was evaluated by measuring an OD value at a wavelength of 600 nm using a spectrophotometer.

In addition, concentrations of the excreted beta-carotene, retinol, and retinal were measured by mixing 0.1 ml of the supernatant, from which the cells were removed after completing the culture, with 0.9 ml of acetone (Sigma) comprising 4% BHT, and then quantitatively analyzing the same using HPLC instrument.

The analyzed OD values and the concentrations of retinoid and beta-carotene are as shown in Table 4, and the retinoid concentrations are schematized and shown in FIG. 1.

**[Table 4]**

| Strain | OD (600nm) | Retinol (ug/L) | Retinal (ug/L) | β-carotene (ug/L) |
|---|---|---|---|---|
| CC08-2050 (control) | 80 | 8060 | 1753 | N.D |
| CJ2327 | 74 | 16773 | 3634 | N.D |
| CJ2328 | 72 | 19319 | 3961 | N.D |
| CJ2329 | 77 | 18543 | 3901 | N.D |

As shown in the above results, when CRH1 (YALl0C09680g), CRH1 (YALl0E24673g), and CRH2 (YALl0B15510g) were weakened in the retinoid-producing *Yarrowia* strain, retinol/retinal excreted from the cells increased by 2.1-times/2.1-times, 2.4-times/2.3-times, and 2.3-times/2.2-times, respectively, as compared to the control group. In addition, the above results suggest that only retinoid is selectively excreted even though beta-carotene is produced in the retinoid-producing microorganisms, in which CRH1, CRH2, or a combination thereof is weakened.

Based on the above description, it will be understood by those skilled in the art that the present disclosure may be implemented in a different specific form without changing the technical spirit or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

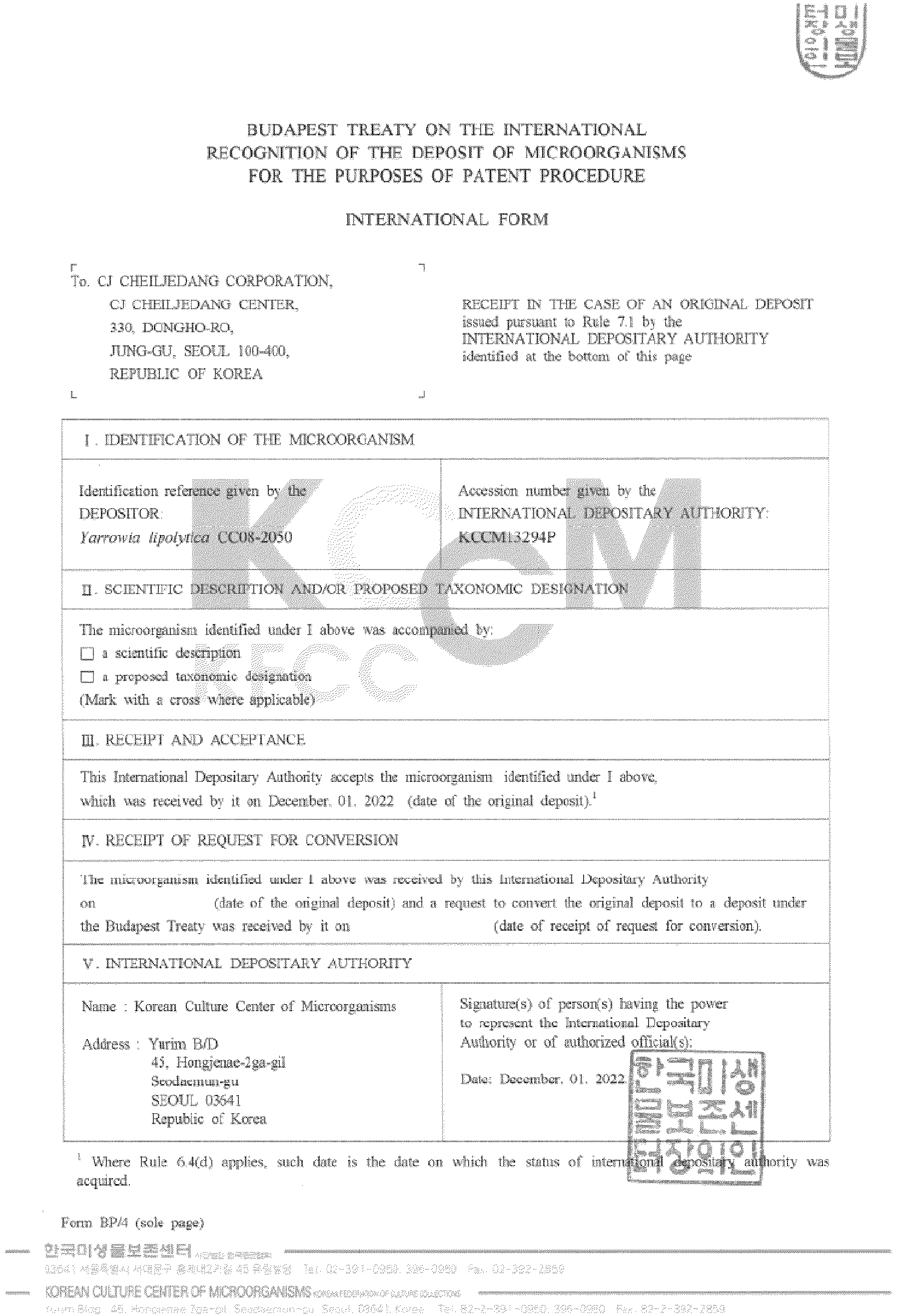
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

To. CJ CHEILJEDANG CORPORATION,
CJ CHEILJEDANG CENTER,
330, DONGHO-RO,
JUNG-GU, SEOUL 100-400,
REPUBLIC OF KOREA

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM

Identification reference given by the
DEPOSITOR:
Yarrowia lipolytica CC08-2050 | Accession number given by the
INTERNATIONAL DEPOSITARY AUTHORITY:
KCCM13294P

II. SCIENTIFIC DESCRIPTION AND/OR PROPOSED TAXONOMIC DESIGNATION

The microorganism identified under I above was accompanied by:
☐ a scientific description
☐ a proposed taxonomic designation
(Mark with a cross where applicable)

III. RECEIPT AND ACCEPTANCE

This International Depositary Authority accepts the microorganism identified under I above,
which was received by it on December. 01. 2022 (date of the original deposit).¹

IV. RECEIPT OF REQUEST FOR CONVERSION

The microorganism identified under I above was received by this International Depositary Authority
on (date of the original deposit) and a request to convert the original deposit to a deposit under
the Budapest Treaty was received by it on (date of receipt of request for conversion).

V. INTERNATIONAL DEPOSITARY AUTHORITY

Name : Korean Culture Center of Microorganisms

Address : Yurim B/D
45, Hongjenae-2ga-gil
Seodaemun-gu
SEOUL 03641
Republic of Korea | Signature(s) of person(s) having the power
to represent the International Depositary
Authority or of authorized official(s):

Date: December. 01. 2022.

¹ Where Rule 6.4(d) applies, such date is the date on which the status of international depositary authority was
acquired.

Form BP/4 (sole page)

한국미생물보존센터 [?]
03641 서울특별시 서대문구 홍제내2가길 45 유림빌딩 Tel. 02-391-0950, 396-0950 Fax. 02-392-2859
KOREAN CULTURE CENTER OF MICROORGANISMS [?]
Yurim Bldg. 45, Hongjenae 2ga-gil Seodaemun-gu Seoul, 03641, Korea Tel. 82-2-391-0950, 396-0950 Fax. 82-2-392-2859

## Claims

1. A microorganism of the *Yarrowia* sp. having a retinoid production ability, wherein the activity of chitin transglycosylase is weakened.

2. The microorganism of claim 1, wherein the chitin transglycosylase is CRH1 protein, CRH2 protein, or a combination thereof.

3. The microorganism of claim 1, wherein the chitin transglycosylase is any one or more selected from the group consisting of a polypeptide comprising a sequence having 60% or more identity to an amino acid sequence of SEQ ID NO: 1 and exhibiting the activity of chitin transglycosylase, a polypeptide comprising a sequence having 60% or more identity to an amino acid sequence of SEQ ID NO: 2 and exhibiting the activity of chitin transglycosylase, and a polypeptide comprising a sequence having 60% or more identity to an amino acid sequence of SEQ ID NO: 3 and exhibiting the activity of chitin transglycosylase.

4. The microorganism of claim 1, wherein the chitin transglycosylase is derived from *Yarrowia lipolytica.*

5. The microorganism of claim 1, wherein the chitin transglycosylase is encoded by SEQ ID NO: 4, SEQ ID NO: 5, SEQ ID NO: 6, or a polynucleotide sequence having 60% or more identity thereto.

6. The microorganism of claim 1, wherein the microorganism has an increased retinoid excretion ability, as compared to a microorganism of the *Yarrowia* sp. having a retinoid production ability, in which the activity of chitin transglycosylase is not weakened.

7. The microorganism of claim 1, wherein the microorganism of the *Yarrowia* sp. is *Yarrowia lipolytica.*

8. The microorganism of claim 1, wherein the retinoid comprises any one selected from the group consisting of retinol, retinal, retinoic acid, and retinyl ester.

9. A method of producing retinoids, the method comprising the step of culturing the microorganism of any one of claims 1 to 8 in a medium.

10. The method of claim 9, comprising the step of recovering retinoids from the cultured medium or microorganism.

11. The method of claim 9, wherein cell disruption of microorganisms or dodecane as a solvent is not used, when retinoids are extracted.

12. The method of claim 9, wherein the retinoid comprises any one selected from the group consisting of retinol, retinal, retinoic acid, and retinyl ester.

13. A method of preparing a microorganism of the *Yarrowia* sp. having a retinoid production ability, the method comprising the step of weakening the activity of chitin transglycosylase in the microorganism of the *Yarrowia* sp. having the retinoid production ability.

14. A method of increasing excretion of retinoids, the method comprising the step of weakening the activity of chitin transglycosylase in a microorganism of the *Yarrowia* sp. having a retinoid production ability.

15. A composition for producing retinoids, the composition comprising any one or more of a microorganism of the *Yarrowia* sp. having a retinoid production ability, in which the activity of chitin transglycosylase is weakened, and a culture thereof.

16. Use of a microorganism of the *Yarrowia* sp. having a retinoid production ability, in which the activity of chitin transglycosylase is weakened, in producing retinoids.
